# EUROPEAN PATENT SPECIFICATION

(11) **EP 2 921 126 B1**
(45) Date of publication and mention of the grant of the patent: **01.11.2017**
(21) Application number: 13855396.1
(22) Date of filing: 12.09.2013
(51) Int. Cl.: A61B 18/20

(54) **LASER ABLATION DEVICE**
LASERABLATIONSVORRICHTUNG
DISPOSITIF D'ABLATION PAR LASER

(30) Priority: 13.11.2012 JP 2012249519
(43) Date of publication of application: 23.09.2015
(73) Proprietor: Olympus Corporation, Tokyo 192-8507 (JP)
(72) Inventor: EBATA, Sadao, Hachioji-shi, Tokyo 192-8507 (JP)
(74) Representative: Schicker, Silvia
(86) International application number: PCT/JP2013/074632
(87) International publication number: WO 2014/077022

(56) References cited:
- EP-A2- 1 118 311
- WO-A2-2008/002625
- JP-A- 2001 190 565
- JP-A- 2003 079 752
- JP-A- 2003 265 498
- JP-A- 2010 268 961
- JP-A- 2012 037 883
- US-A1- 2004 231 682
- US-A1- 2006 029 341
- US-A1- 2008 161 648
- US-B1- 6 485 413

## Description

### {Technical Field}

The present invention relates to a laser ablation device as defined in claim 1.

### {Background Art}

In the conventional art, a laser ablation catheter with which laser light is radiated onto affected tissue from an insertion portion that emits laser light having high-density energy, thus cauterizing the affected tissue, has been known (for example, PTL 1). The laser ablation catheter is used mainly to perform arrhythmia treatment and has the advantage, for patients, that only a minimum region into which the laser ablation catheter can be inserted needs to be incised, thereby making it possible to cauterize an affected area, which allows minimally invasive surgery to be performed.

PTL 2 discloses an apparatus for laser light irradiation to a living body comprising a casing which serves as holding means and a hollow guide, which is disposed within the casing in such a manner that it is rotatable around the turning center axis and is held in a support cylinder by front and rear bearings, the casing having a removable cover in the rear of a grip body. An optical fiber is inserted into the casing through the cover of the casing, and further inserted inside the tubular guide. A holding member is secured integrally within the tip end of the tubular guide, and the front end of the optical fiber is held in a groove which is formed on the outer periphery of the holding member. The optical fiber is optically coupled with laser light supplying means, that may include a laser light generator and pulsating means. According to PTL 2, by changing the manner of reflecting of the laser light on the inner surface by selecting an appropriate reflecting cylinder among the plurality of reflecting cylinders depending upon the situation, the irradiating energy distribution of the laser light with respect to the tissue can be changed. The energy of the laser light which is incident upon the tissue can also be changed even if the energy of the laser light emitted from the front end of the optical fiber is made constant.

PTL 3 discloses a handheld photocosmetic device for performing fractional treatment of a tissue, comprising a housing, an EMR source disposed in the housing, and an EMR delivery path that is located within the housing and optically coupled to the light source, wherein the EMR delivery path is configured to apply EMR generated by the EMR source to a plurality of discrete locations located within a treatment area of the tissue, and wherein a total area of the plurality of discrete locations is less than the treatment area. The EMR delivery path may comprise an optical scanner, which includes at least one optical fiber having an input port adapted to receive EMR from the EMR source and having an output port through which EMR can be delivered to the locations. According to PTL 3, a light guide can be made from a bundle of optical fibers doped with ions of rear earth metals; the active ions inside the light guide core can act as fluorescent (or super fluorescent) converters to provide desired spatial modulation and spectrum conversion. The optical fibers can be wrapped around the EMR source in order to couple light into the optical fibers.

PTL 4 discloses an optical scanning device including a bundle of optical fibers, connected to an optical energy source, capable of outputting sufficient energy for performing a dermatological procedure. For example, the optical energy source may include a fiber coupled laser source, such as a laser diode, whose operation may be controlled by a controller. The laser diode may be tunable and may be provided with drivers and collimators as needed. The optical energy source is not limited to coherent light and may include a source of non-coherent light (e.g., flash lamp) as well. The bundle of optical fibers may include, without limitation, single mode or multi-mode fibers, which may or may not be doped.

PTL 5 discloses an imaging system for performing forward scanning imaging for application to therapeutic and diagnostic devices used in medical procedures. The imaging system includes forward directed optical coherence tomography (OCT), and non-retroreflected forward scanning OCT. Also interferometric imaging and ranging techniques and fluorescent, Raman, two-photon, and diffuse wave imaging can be used. The forward scanning mechanisms include a cam attached to a motor, pneumatic devices, a pivoting device, piezoelectric transducers, electrostatic driven slides for substantially transverse scanning. Counter-rotating prisms and offset lenses are used for arbitrary scanning. One or more optical fibers can be positioned within the scalpel blade itself with the tip of the fiber located at the tip or along the edge of the scalpel blade. With high densities of fibers, imaging can be performed whereas with lower numbers of fibers, isolated depth information can be acquired. Single axial scans can be performed which can provide depth ranging information of the tissue prior to scalpel blade insertion. If the fibers are in near contact with the specimen or sample, the lenses to focus the light may be low power or may not be needed at all.

PTL 6 discloses a system and method of ablation laser-machining, that includes the steps of generating pulses at 1 to 50 MHz by one or more semiconductor-chip laser diodes, each pulse having a pulse-duration less than three picoseconds, directing a less than 1 square mm beam of the pulses to a work-piece with an ablating pulse-energy-density; and scanning the beam with a power-driven scanner to ablate a scanned area at least 25 times larger than the beam area. According to PTL 6, when the ablation is part of a surgical procedure, the ablating pulse-energy-density is between 1 and 10 times the ablation threshold, and more preferably about 1 to 3 times the ablation threshold to minimize collateral damage. The beam is scanned in at least one, and preferably two or more directions, and can be scanned in a spiral. The beam is preferably scanned to travel at a speed of at least one meter per second on the work-piece.

### {Citation List}

### {Patent Literature}

{PTL 1} Japanese Unexamined Patent Application, Publication No. Hei 7-8502
{PTL 2} European Patent Application, Publication No. 1 118 311 A2;
{PTL 3} International Patent Application, Publication No. 2008/002625 A2
{PTL 4} US Patent Application, Publication No.2006/029341 A1
{PTL 5} US Patent No. 6 485 413 B1
{PTL 6} US Patent Application, Publication No. 2004/231682 A1

### {Summary of Invention}

### {Technical Problem}

However, with the above-described laser ablation catheter, because laser light having high energy is radiated in a linear fashion, although the laser cauterization performance at the affected area is high, there is a risk that excessive laser radiation may cause damage to or perforation of normal tissue. Thus, an operator manually vibrates an insertion portion and operates the laser ablation catheter so as to prevent laser light from being locally and excessively radiated; however, this requires a highly-skilled operation, thus imposing a burden on the operator. Furthermore, particularly in a narrow space in the pericardium, it is difficult to allow manipulations performed by the operator at an insertion-portion base end to be transferred to an insertion-portion distal end, and manipulations that depend on the operator are limited. Furthermore, although the amount of light, the radiation region, and the radiation time are specified for laser radiation for treatment, because the density of laser radiation differs depending on the manipulation route, uneven radiation occurs, so that there is a risk that the desired radiation cannot be performed.

The present invention has been made in view of the above-described circumstances, and an object thereof is to provide a laser ablation device that prevents local excessive laser radiation and that performs uniform laser radiation in a target treatment region.

### {Solution to Problem}

In order to achieve the above-described object, the present description provides the following solutions.

According to one aspect, the present description provides a laser ablation device including: a light source that emits laser light for cauterizing an affected area; a fiber that is provided in an insertion portion being inserted into the body and being a long bendable pipe conduit and that guides the laser light emitted from the light source to radiate the laser light from an insertion-portion distal end; and a first drive unit that is provided on the fiber and that vibrates the fiber with a first period.

According to this aspect, laser light emitted from the light source is guided by the fiber, which is provided in the insertion portion, and the fiber emits the laser light from the insertion-portion distal end. Although the laser light emitted from the insertion-portion distal end, specifically, from the emitting end of the fiber, is radiated onto the affected area in a linear fashion, because the laser light is periodically vibrated by the first drive unit, the laser light can be radiated uniformly onto a predetermined area without being radiated onto a fixed point. Therefore, it is possible to perform uniform laser radiation in a target treatment region and also to prevent local excessive laser radiation.

In the above-described aspect, the laser ablation device further includes a second drive unit that vibrates the fiber with a second period.

By doing so, it is possible to expand a laser-light radiation area and to uniformly radiate laser light onto a wider region. Furthermore, it is possible to set the first period and the second period to different periods or also to the same period.

In the above-described aspect, the amplitude produced by the second drive unit is larger than the amplitude produced by the first drive unit.

By doing so, because the first drive unit can perform relatively-fine laser-light radiation, and the second drive unit can perform relatively-rough radiation, it is possible to evenly perform uniform laser-light radiation on an affected area while expanding the laser-light radiation area as a whole.

In the above-described aspect, it is preferable that the first drive unit be provided closer to a distal end of the fiber than the second drive unit; and the second period be longer than the first period.

Furthermore, in the above-described invention, it is preferable that the first drive unit be provided closer to a distal end of the fiber than the second drive unit; and the second period be a period n times (n is an integer) the first period.

By doing so, laser light can be radiated onto the affected area more uniformly.

In the above-described aspect, it is preferable that the fiber be made to perform rotational motions by the first drive unit and the second drive unit; and the number of rotations of the fiber due to the first drive unit be faster than the number of rotations of the fiber due to the second drive unit.

By doing so, laser light can be radiated onto the affected area more uniformly.

Note that the first drive unit and the second drive unit allow the fiber to perform a resonant motion, raster scanning, spiral scanning, and scanning obtained by combining different types of scanning. In this way, by allowing the resonant motion, the electro-mechanical conversion efficiency is enhanced, thus making it possible to reduce the voltage required to perform predetermined treatment.

Furthermore, because the shape of laser radiation area can be changed by changing the scanning method to raster scanning, spiral scanning, or the like, it is possible to achieve a reduction in radiation time and a reduction in the number of times radiation is performed, by selecting an appropriate scanning method in accordance with the shape of a treatment target.

Furthermore, by further including one or more other drive units for periodically vibrating the fiber, it is possible to radiate laser light onto the affected area more uniformly and to achieve a reduction in radiation time and a reduction in the number of times radiation is performed.

### {Advantageous Effects of Invention}

According to the present invention, an advantageous effect is afforded in that it is possible to prevent local excessive laser radiation and to allow uniform laser radiation in a target treatment region.

### {Brief Description of Drawings}

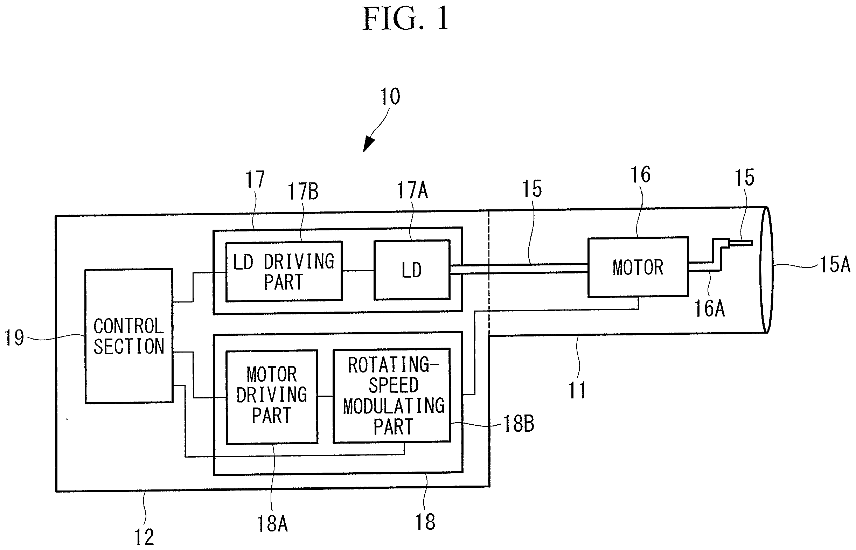

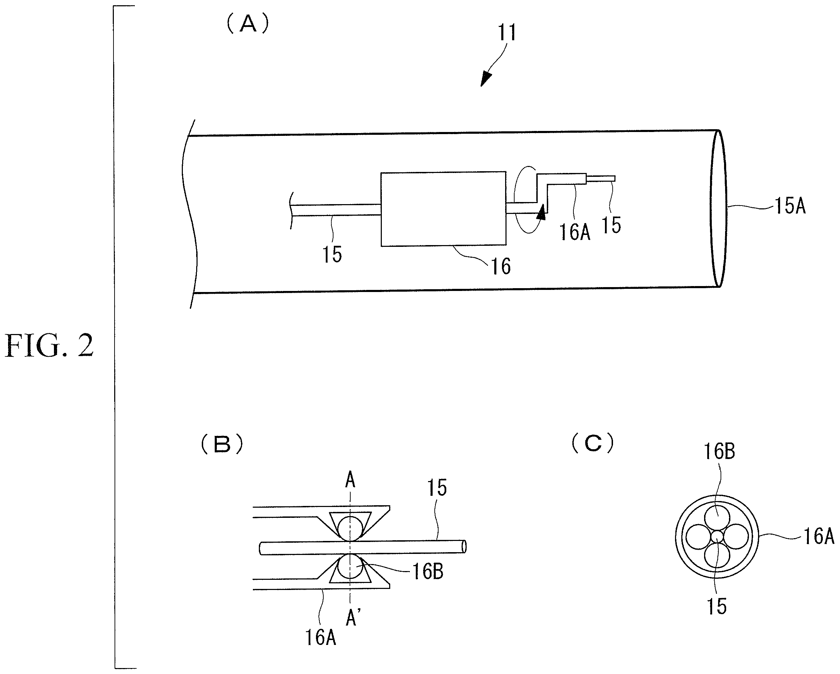

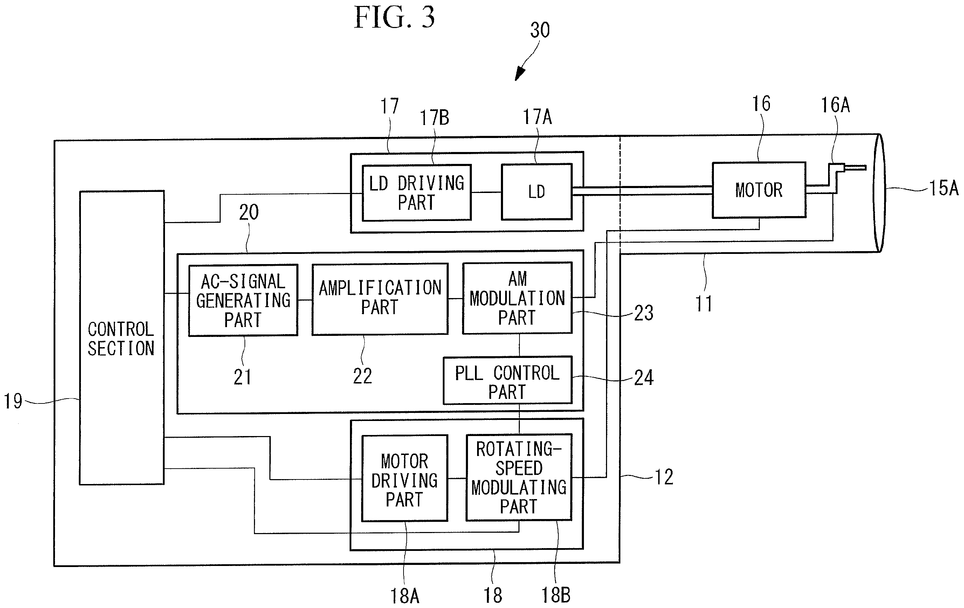

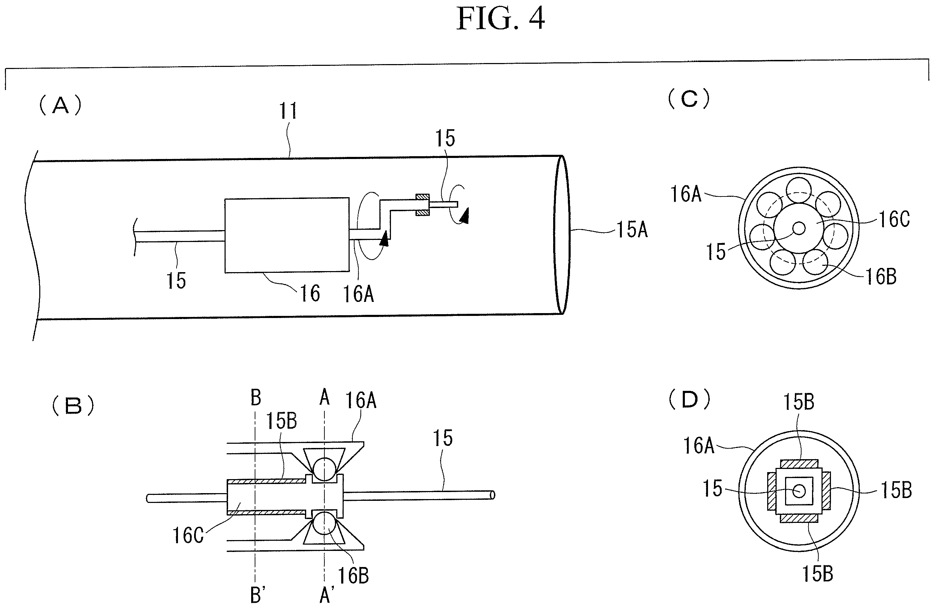

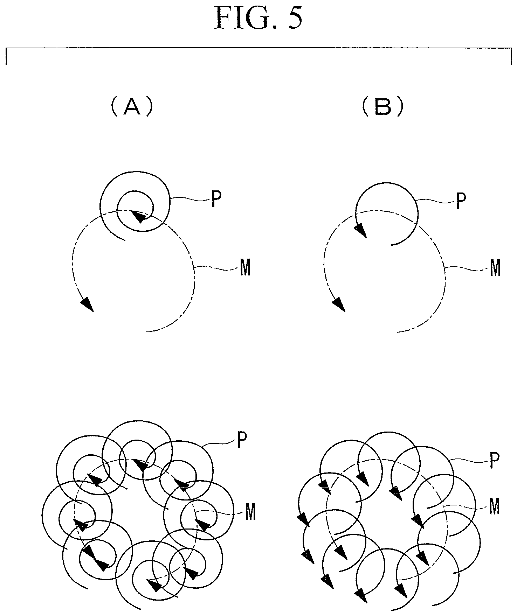

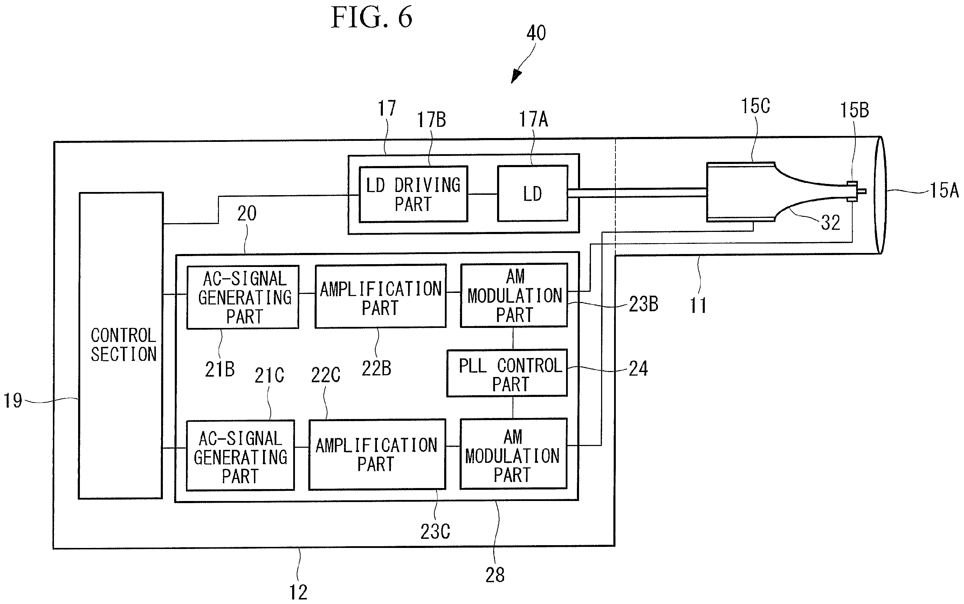

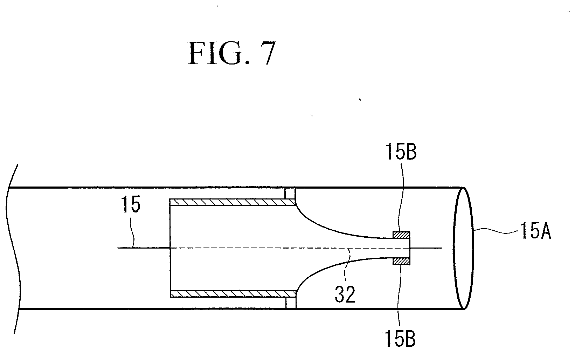

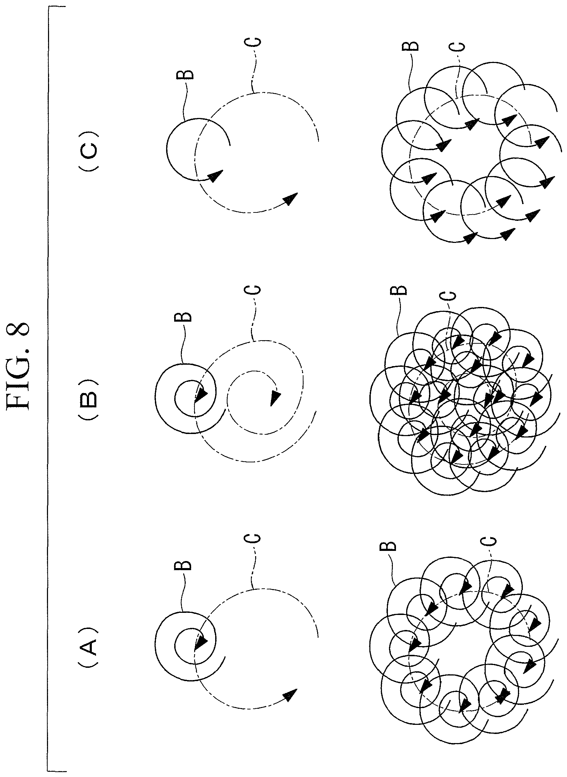

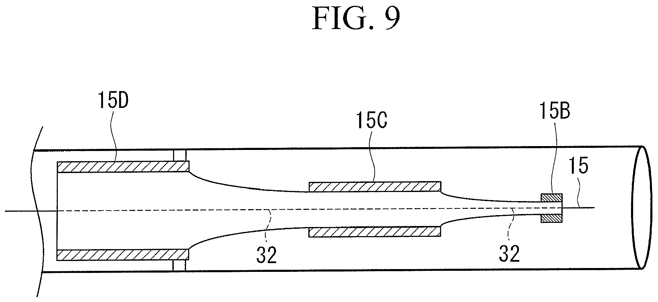

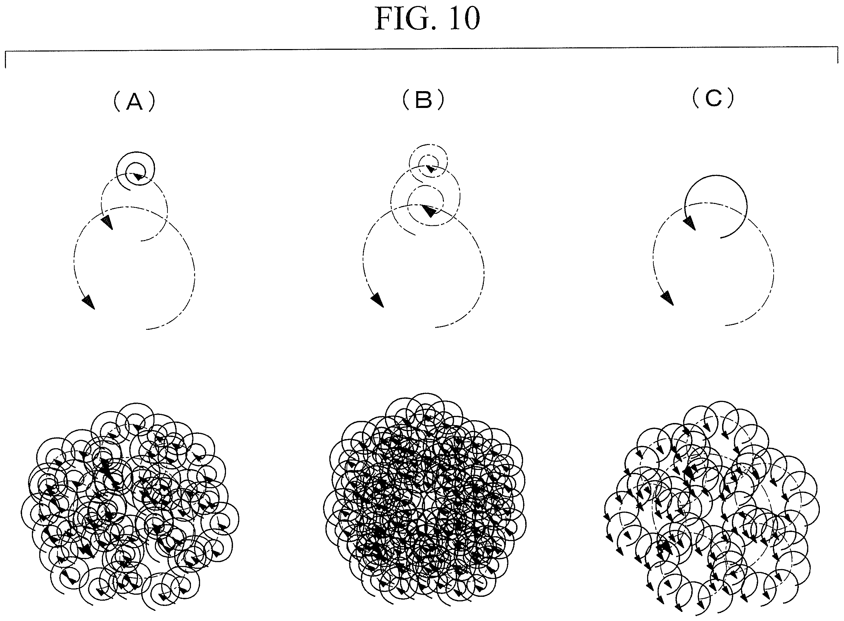

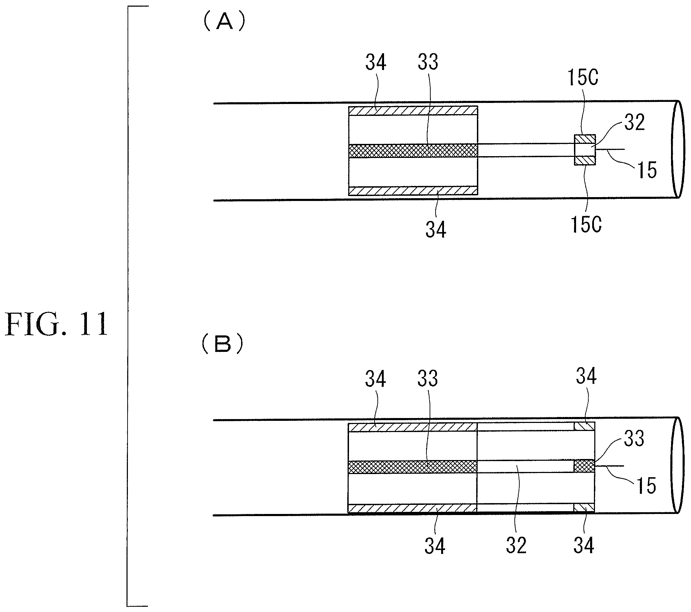

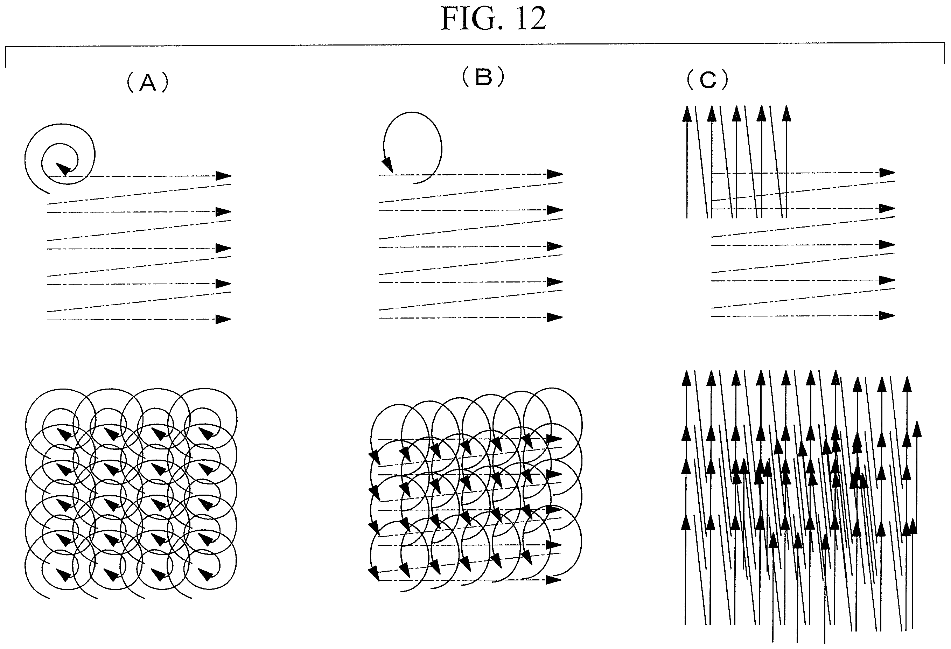

{Fig. 1} Fig. 1 is a view showing the overall configuration of a laser ablation device according to a first embodiment of the present description.
{Figs. 2A to 2C} Figs. 2A to 2C show an insertion portion of the laser ablation device according to the first embodiment of the present description : Fig. 2A is a view showing the overall insertion portion; Fig. 2B is a view showing a state in which a shaft and a fiber are fixed; and Fig. 2C is a cross-sectional view cut along the line A-A' in Fig. 2B.
{Fig. 3} Fig. 3 is a view showing the overall configuration of a laser ablation device according to a second embodiment of the present description.
{Figs. 4A to 4D} Figs. 4A to 4D show an insertion portion of the laser ablation device according to the second embodiment of the present description : Fig. 4A is a view showing the overall insertion portion; Fig. 4B is a view showing a state in which a shaft and a fiber are fixed; Fig. 4C is a cross-sectional view cut along the line A-A' in Fig. 4B; and Fig. 4D is a cross-sectional view cut along the line B-B' in Fig. 4B.
{Figs. 5A and 5B} Figs. 5A and 5B show example laser-light radiation trajectories produced by the fiber of the laser ablation device according to the second embodiment of the present description.
{Fig. 6} Fig. 6 is a view showing the overall configuration of a laser ablation device according to a third embodiment of the present description.
{Fig. 7} Fig. 7 is a view showing an insertion portion of the laser ablation device according to the third embodiment of the present description.
{Figs. 8A to 8C} Figs. 8A to 8C show example laser-light radiation trajectories produced by a fiber of the laser ablation device according to the third embodiment of the present description.
{Fig. 9} Fig. 9 is a view showing an insertion portion of a laser ablation device according to a modification of the third embodiment of the present description.
{Figs. 10A to 10C} Figs. 10A to 10C show example laser-light radiation trajectories produced by a fiber of the laser ablation device according to the modification of the third embodiment of the present description.
{Figs. 11A and 11B} Figs. 11A and 11B show other examples of insertion portions of laser ablation devices according to the present description.
{Figs. 12A to 12C} Figs. 12A to 12C show example laser-light radiation trajectories produced by fibers of the laser ablation devices shown in Figs. 11A and 11B.

### {Description of Embodiments}

### First Embodiment

A laser ablation device 10 according to a first embodiment of the present description will be described below with reference to the drawings. The laser ablation device 10 of this embodiment radiates laser light from an insertion portion, to be described later, onto affected tissue to cauterize the affected tissue, thereby performing treatment for arrhythmia etc., and includes an insertion portion 11 and a main portion 12.

As shown in Figs. 1 and 2, the insertion portion 11 to be inserted into the body of patients is a long bendable pipe conduit and includes a fiber 15 that guides laser light emitted from a light source, to be described later, and that radiates the laser light from an insertion-portion distal end and a motor 16 that is provided on the fiber 15 to vibrate the fiber 15 with a predetermined period. Specifically, the fiber 15 is provided integrally with a shaft 16A of the motor 16 and guides laser light while rotating in conjunction with rotation of the motor 16.

Specifically, the shaft 16A has a hollow structure, and the fiber 15 passes through the shaft 16A. The shaft 16A of the motor 16 has a bent portion, so that the output of the motor 16 is made to be eccentric with respect to the axis of rotation. As shown in Fig. 2C, four ball bearings 16B are disposed in a distal end of the shaft 16A at equal-spaced intervals, the fiber 15 is in contact with the shaft 16A via the ball bearings 16B, and thus the fiber 15 is fixed to the shaft 16A. A lens 15A through which laser light emitted from an emitting end of the fiber 15 is transmitted is provided on a distal end surface of the insertion portion 11.

The main portion 12 includes a light source section 17, a vibration control section 18 that controls the vibration of the fiber 15, and a control section 19 that controls the light source section 17 and the vibration control section 18.

The light source section 17 includes an LD (laser diode) 17A that serves as the light source, which emits laser light for cauterizing an affected area, and an LD driving part 17B that drives the LD 17A.

The vibration control section 18 has a motor driving part 18A that rotationally drives the motor 16 and a rotating-speed modulating part 18B that appropriately modulates the rotating speed of the motor 16.

The operation of the thus-configured laser ablation device 10 will be described below.

The distal end of the insertion portion 11 of the laser ablation device 10 is inserted up to the vicinity of an affected area. In this state, when power is supplied from the LD driving part 17B to the LD 17A based on a control signal sent from the control section 19, laser light is emitted from the LD 17A and enters an incident end of the fiber 15 that is located at a base end of the insertion portion 11. The laser light is guided by the fiber 15 to the distal end of the fiber 15 and is radiated from the emitting end of the fiber 15 onto the affected area via the lens 15A, which is provided at the distal end of the insertion portion 11.

At this time, the fiber 15 is provided integrally with the shaft 16A of the motor 16 so as to guide the laser light while rotating in conjunction with rotation of the motor 16. Furthermore, because the shaft 16A of the motor 16 makes the output of the motor 16 eccentric with respect to the axis of rotation, when the motor 16 is rotationally driven by the motor driving part 18A, the laser light emitted from the fiber 15 is radiated onto the affected area while tracing a circular trajectory corresponding to the eccentric position of the shaft 16A.

As described above, according to this embodiment, rotation of the motor 16 vibrates the fiber 15, which emits laser light, thereby making it also possible to vibrate the laser-light radiation trajectory, thus preventing laser light from being locally radiated onto the affected area and allowing uniform laser-light radiation while expanding the radiation region.

### Second Embodiment

Next, a laser ablation device 30 according to a second embodiment of the present description will be described below with reference to the drawings. In this embodiment, identical reference signs are assigned to the same components as those in the above-described first embodiment, and a description thereof will be omitted. This embodiment mainly differs from the first embodiment in that piezoelectric elements 15B are provided symmetrically in four directions around the axis of the output end of the shaft 16A, as shown in Fig. 3.

Therefore, the main portion 12 further includes a piezoelectric-element control section 20 that controls the piezoelectric elements, and the control section 19 controls the light source section 17, the vibration control section 18, and the piezoelectric-element control section 20.

The piezoelectric-element control section 20 includes an AM modulation part 23 that supplies electric power to the piezoelectric elements 15B, a PLL control part 24 that adjusts the phases of modulated signals output from the AM modulation part 23 and the number of rotations of the motor 16, an AC-signal generating part 21 that generates AC signals to be supplied to the AM modulation part 23, and an amplification part 22 that amplifies the AC signals output from the AC-signal generating part 21.

As shown in Figs. 4A to 4D, the fiber 15 is provided in the hollow shaft 16A, and the distal end of the fiber 15 is fixed to the shaft 16A by ball bearings 16B that are provided via an elastic member 16C. Contact points of the ball bearings 16B are located at the position of a node of a vibration of the elastic member. Because the piezoelectric elements 15B are provided symmetrically in four directions around the axis of the fiber 15 via the elastic member 16C and are composed of X-axis-driving piezoelectric elements and Y-axis-driving piezoelectric elements, the phases of the AC signals supplied from the AC-signal generating part 21 to the X-axis-driving piezoelectric elements and the Y-axis-driving piezoelectric elements are shifted by 90 degrees. Furthermore, the modulated signals output from the AM modulation part 23 and the rotating speed of the motor 16 are individually controlled by the PLL control part 24 so as to establish a relationship between frequency division and multiplication.

The operation of the thus-configured laser ablation device will now be described.

AC signals generated by the AC-signal generating part 21 are amplified by the amplification part 22 and are AM-modulated at the AM modulation part 23. The frequencies of the voltage and the current to be applied to the piezoelectric elements 15B are made to match the resonance frequency of a vibration of the fiber 15. When the modulated signals output from the AM modulation part 23 are supplied to the piezoelectric elements 15B, the piezoelectric elements 15B vibrate due to the piezoelectric effect, thus vibrating the shaft 16A. The vibration is transferred to make the fiber 15 resonate.

In this state, when the LD driving part 17B supplies predetermined power to the LD 17A based on a control signal of the control section 19, the LD 17A emits laser light toward the emitting end of the fiber 15. The emitted laser light is radiated onto an affected area from the insertion-portion distal end via the fiber 15.

At this time, as described above, because the motor 16 is driven, thereby rotating the shaft 16A, and the piezoelectric elements 15B vibrate due to the piezoelectric effect, thereby vibrating the distal end of the shaft 16A, light radiated from the distal end of the insertion portion 11 traces a radiation trajectory obtained by superposing a vibration produced by the motor and a vibration produced by the piezoelectric elements 15B.

Figs. 5A and 5B show example laser-light radiation trajectories produced by the fiber 15. Fig. 5A shows an example radiation trajectory in the case where, by setting the amplitude of a vibration produced by the piezoelectric elements 15B smaller than the amplitude of a vibration produced by the motor 16, the motor 16 roughly moves the laser light at the same time as the piezoelectric elements 15B finely move the laser light. Fig. 5A shows an example laser-light radiation trajectory in the case where the piezoelectric elements 15B vibrate the fiber 15 in a spiral pattern, and Fig. 5B shows an example laser-light radiation trajectory in the case where the piezoelectric elements 15B vibrate the fiber 15 in a circular trajectory.

In this way, according to this embodiment, by superposing the vibration produced by rotational motion of the motor 16 and the vibration produced by the piezoelectric elements 15B, it is possible to prevent the laser light from being locally radiated and also to allow more uniform laser-light radiation, which prevents damage to tissue other than the affected area. Because it is possible to avoid fixed-point radiation and to allow area radiation, the therapeutic dose can be visually perceived with observation optics, such as an endoscope.

Note that the number of rotations, the rotating speed, and the direction of rotation of the motor may be desirably set, and the amplitude of the motor may be different from or may be the same as the amplitude of the piezoelectric elements. Furthermore, in this embodiment, although a description has been given of a case in which the frequencies of the voltage and the current to be applied to the piezoelectric elements 15B are made to match the resonance frequency of the vibration of the fiber 15, the frequencies are not necessarily resonant and may be non-resonant.

### Third Embodiment

Next, a laser ablation device 40 according to a third embodiment of the present description will be described with reference to the drawings. In this embodiment, identical reference signs are assigned to the same components as those in the above-described second embodiment, and a description thereof will be omitted. This embodiment mainly differs from the second embodiment in that piezoelectric elements 15C are provided instead of the motor 16, as shown in Figs. 6 and 7.

Specifically, the fiber 15 is provided with an elastic member 32 for supporting the piezoelectric elements 15B and the piezoelectric elements 15C. The piezoelectric elements 15B are provided symmetrically in four directions at a distal end of the elastic member 32, and the piezoelectric elements 15C are provided symmetrically in four directions at a base end thereof.

Therefore, the main portion 12 includes, instead of the piezoelectric-element control section 20, a piezoelectric-element control section 28 that controls the piezoelectric elements 15B and the piezoelectric elements 15C.

The piezoelectric-element control section 28 includes AM modulation parts 23B and 23C that supply electric power to the piezoelectric elements 15B and 15C, respectively, a PLL control part 24 that individually adjusts the phases of modulated signals output from the AM modulation parts 23B and 23C, AC-signal generating parts 21B and 21C that generate AC signals to be supplied to the AM modulation parts 23B and 23C, and amplification parts 22B and 22C that amplify the AC signals output from the AC-signal generating parts 21B and 21C.

AC signals generated by the AC-signal generating part 21B are amplified at the amplification part 22B and are AM-modulated at the AM modulation part 23B. Similarly, AC signals generated by the AC-signal generating part 21C are amplified at the amplification part 22C and are AM-modulated at the AM modulation part 23C. Although the modulated signals output from the AM modulation part 23B and the AM modulation part 23C have different frequencies, they are controlled at the PLL control part 24 so as to establish a relationship between frequency division and multiplication. Furthermore, the frequencies of the voltage and the current to be applied to the piezoelectric elements 15B are made to match the resonance frequency at the distal end portion of the elastic member 32, and the frequencies of the voltage and the current to be applied to the piezoelectric elements 15C are made to match the resonance frequency of the fiber 15.

The operation of the thus-configured laser ablation device will now be described. Modulated signals output from the AM modulation part 23B and the AM modulation part 23C are supplied to the piezoelectric elements 15B and 15C, respectively, and the piezoelectric elements 15B and 15C vibrate due to the piezoelectric effect based on the modulated signals. The vibrations are transferred via the elastic member 32 to vibrate the fiber 15.

In this state, when the LD driving part 17B supplies predetermined power to the LD 17A based on a control signal output from the control section 19, the LD 17A emits laser light toward the incident end of the fiber 15. The emitted laser light is emitted from the distal end of the insertion portion 11 via the fiber 15.

At this time, because the piezoelectric elements 15B and 15C vibrate the fiber 15, the laser light emitted from the distal end of the insertion portion 11 traces a radiation trajectory obtained by superposing a vibration produced by the piezoelectric elements 15B and a vibration produced by the piezoelectric elements 15C.

Figs. 8A to 8C show example laser-light radiation trajectories produced by the fiber 15. Figs. 8A to 8C show example radiation trajectories in the case where, by setting the amplitude of a vibration produced by the piezoelectric elements 15B smaller than the amplitude of a vibration produced by the piezoelectric elements 15C, the piezoelectric elements 15C roughly move the laser light at the same time as the piezoelectric elements 15B finely move the laser light. Fig. 8A shows an example laser-light radiation trajectory in the case where the piezoelectric elements 15B vibrate the fiber 15 in a spiral pattern at the same time as the piezoelectric elements 15C vibrate the fiber 15 in a circular trajectory, and Fig. 8B shows an example laser-light radiation trajectory in the case where the piezoelectric elements 15B vibrate the fiber 15 in the same way as in Fig. 8A, and the piezoelectric elements 15C vibrate the fiber 15 in a spiral pattern. Fig. 8C shows an example laser-light radiation trajectory in the case where both the piezoelectric elements 15B and 15C vibrate the fiber 15 in a circular trajectory.

In this way, according to this embodiment, the vibration produced by the piezoelectric elements 15B and the vibration produced by the piezoelectric elements 15C are transferred to the fiber 15 via the elastic member 32, and the vibration produced by the piezoelectric elements 15B and the vibration produced by the piezoelectric elements 15C are superposed, thereby making it possible to prevent the laser light from being locally radiated and also to allow more uniform laser-light radiation, which prevents damage to tissue other than the affected area. Because it is possible to avoid fixed-point radiation and to allow area radiation, the therapeutic dose can be visually perceived with observation optics, such as an endoscope. Because the variable range of the radiation region is wide, it is possible to respond flexibly to different treatment regions.

### Modification of Third Embodiment

Next, a laser ablation device according to a modification of the third embodiment of the present description will be described with reference to the drawings. In this modification, identical reference signs are assigned to the same components as those in the above-described third embodiment, and a description thereof will be omitted. This embodiment mainly differs from the third embodiment in that a so-called three-stage structure in which piezoelectric elements are provided at three places in the axial direction of the elastic member 32 is built, as shown in Fig. 9.

Specifically, the fiber 15 is provided with an elastic member 32 for supporting the piezoelectric elements 15B, the piezoelectric elements 15C, and piezoelectric elements 15D. The elastic member 32 has the piezoelectric elements 15B provided symmetrically in four directions at the distal end, the piezoelectric elements 15C provided symmetrically in four directions closer to the base end than the piezoelectric elements 15B, and the piezoelectric elements 15D provided symmetrically in four directions at the base end.

Therefore, as in the above-described third embodiment, the main portion includes, instead of the piezoelectric-element control section 20, a piezoelectric-element control section 28 that controls the piezoelectric elements 15B, the piezoelectric elements 15C, and the piezoelectric elements 15D, and the piezoelectric-element control section 28 includes AM modulation parts that supply electric power to the piezoelectric elements 15B, 15C, and 15D, a PLL control part that individually adjusts the phases of modulated signals output from the AM modulation parts, AC-signal generating parts that generate AC signals to be supplied to the AM modulation parts, and amplification parts that amplify the AC signals output from the AC-signal generating parts.

Figs. 10A to 10C show example laser-light radiation trajectories produced by the fiber 15 in the case where the piezoelectric elements 15B, 15C, and 15D are provided at three places in the axial direction of the fiber, as described above. Figs. 10A to 10C show example radiation trajectories in the case where, by setting the amplitude of a vibration produced by the piezoelectric elements that are provided closer to the distal end of the fiber 15 to be smaller, the piezoelectric elements that are provided closer to the base end roughly move the laser light at the same time as the piezoelectric elements that are provided closer to the distal end finely move the laser light. In particular, Fig. 10A shows an example laser-light radiation trajectory in the case where the piezoelectric elements 15B vibrate the fiber 15 in a spiral pattern at the same time as the piezoelectric elements 15C and 15D vibrate the fiber 15 in a circular trajectory. Fig. 10B shows an example laser-light radiation trajectory in the case where the piezoelectric elements 15D vibrate the fiber 15 in a circular trajectory at the same time as the piezoelectric elements 15B and 15C vibrate the fiber 15 in a spiral pattern. Fig. 10C shows an example laser-light radiation trajectory in the case where all of the piezoelectric elements 15B, 15C, and 15D vibrate the fiber 15 in a circular trajectory.

In this way, according to this embodiment, the vibrations produced by the piezoelectric elements 15B, 15C, and 15D are transferred to the fiber 15 via the elastic member 32, and the vibrations produced by the piezoelectric elements 15B, 15C, and 15D are superposed, thereby making it possible to prevent the laser light from being locally radiated and also to allow more uniform laser-light radiation, which prevents damage to tissue other than the affected area. Because it is possible to avoid fixed-point radiation and to allow area radiation, the therapeutic dose can be visually perceived with observation optics, such as an endoscope. Because the variable range of the radiation region is wide, it is possible to respond flexibly to different treatment regions.

Note that, in the above-described embodiments, although piezoelectric elements are used as a means for producing a vibration, such means is not necessarily limited to the piezoelectric elements and can be electromagnetic vibration elements, for example.

Furthermore, although the third embodiment is provided with a two-stage structure that has a drive unit in which the piezoelectric elements 15B produce a vibration and a drive unit in which the piezoelectric elements 15C produce a vibration, and the modification of the third embodiment is provided with a three-stage structure that has three drive units in each of which the piezoelectric elements produce a vibration, a structure having four or more stages may be provided, and every possible means that can vibrate the fiber, such as motors, piezoelectric elements, and electromagnetic vibration elements, can be used alone or in appropriate combinations, as drive units.

For example, as shown in Figs. 11A and 11B, an electromagnetic vibration element 35 has a permanent magnet 33 that is disposed on the axis of the elastic member 32, which transfers a vibration to the fiber 15, and a coil 34 that is provided so as to surround the permanent magnet 33. When the thus-configured electromagnetic vibration element 35 is used, it is possible to build a structure in which the electromagnetic vibration element 35 is provided closer to the base end of the fiber 15, and the piezoelectric elements 15C are provided closer to the distal end thereof, as shown in Fig. 11A, or a structure in which the electromagnetic vibration element 35 is provided closer to the base end of the fiber 15 and also closer to the distal end thereof, as shown in Fig. 11B.

Then, when current is supplied to the coil, the permanent magnet vibrates due to electromagnetic induction, and this vibration vibrates the distal end of the fiber 15 via the elastic member. Because the electromagnetic vibration element 35 can perform raster scanning, when the piezoelectric elements are provided closer to the distal end of the fiber 15, as shown in Fig. 11A, the raster scanning can be combined with a vibration produced by rotation of the piezoelectric elements, as shown in Figs. 12A and 12B. Furthermore, when the electromagnetic vibration element 35 is provided closer to the base end of the fiber 15 and also closer to the distal end thereof, as shown in Fig. 11B, if both of the electromagnetic vibration elements 35 perform raster scanning, a scan trajectory shown in Fig. 12C can be obtained. In either case, laser light can be prevented from being locally radiated. The present invention is set out in the appended claims. The embodiments, aspects or examples according to the present description that do not fall within the scope of said claims are provided for illustrative purposes only and do not form part of the present invention.

### {Reference Signs List}

- 10, 30, 40: laser ablation device
- 11: insertion portion
- 15: fiber
- 15B: piezoelectric elements
- 15C: piezoelectric elements
- 16: motor
- 16A: shaft
- 17A: light source
- 18: vibration control section
- 19: control section
- 20, 28: piezoelectric-element control section

## Claims

1. A laser ablation catheter (10; 30; 40) comprising:
a light source (17a) adapted to emit laser light for cauterizing an affected area; an insertion portion (11) configured to be inserted into the body and being a long bendable pipe conduit; a fiber (15) that is provided in the insertion portion (11) and adapted to guide the laser light emitted from the light source (17a) to radiate the laser light from the insertion-portion (11) distal end;
a first drive unit (16) that is provided on the fiber (15) and is adapted to vibrate the fiber (15) with a first period;
**characterized by**
a second drive unit (15B) that is adapted to vibrate the fiber (15) with a second period,
wherein the amplitude produced by the second drive unit (15B) is larger than the amplitude produced by the first drive unit (16).

2. A laser ablation device (10; 30; 40) according to claim 1,
wherein the first drive unit (16) is provided closer to the distal end of the fiber (15) than the second drive unit (15B); and
the second period is longer than the first period.

3. A laser ablation device (10; 30; 40) according to claim 1 or 2,
wherein the first drive unit (16) is provided closer to the distal end of the fiber (15) than the second drive unit (15B); and
a length of the second period is n times (n is an integer) a length of the first period.

4. A laser ablation device (10; 30; 40) according to one of claims 1 to 3,
wherein the fiber (15) is adapted to perform rotational motions by the first drive unit (16) and the second drive unit (15B); and
the number of rotations of the fiber (15) due to the first drive unit (16) is higher than the number of rotations of the fiber (15) due to the second drive unit (15B).

5. A laser ablation device (10; 30; 40) according to one of claims 1 to 4, wherein the fiber (15) is adapted to perform a resonant motion.

6. A laser ablation device (10; 30; 40) according to one of claims 1 to 3, wherein the fiber (15) is adapted to perform raster scanning.

7. A laser ablation device (10; 30; 40) according to one of claims 1 to 5, wherein the fiber (15) is adapted to perform spiral scanning.

8. A laser ablation device (10; 30; 40) according to one of claims 1 to 7, further comprising one or more drive units being adapted to vibrate the fiber (15) periodically.

## Patentansprüche

1. Laserablationskatheter (10, 30, 40), der umfasst:
eine Lichtquelle (17a), die dafür geeignet ist, Laserlicht zum Kauterisieren eines betroffenen Bereichs zu emittieren;
einen Einführabschnitt (11), der dafür ausgestaltet ist, in den Körper eingeführt zu werden, und bei dem es sich um eine lange, biegbare Rohrleitung handelt;
eine Faser (15), die in dem Einführabschnitt (11) vorgesehen und dafür geeignet ist, das von der Lichtquelle (17a) emittierte Laserlicht zu leiten, um das Laserlicht von dem distalen Ende des Einführabschnitts (11) auszustrahlen;
eine erste Antriebseinheit (16), die an der Faser (15) vorgesehen und dafür geeignet ist, die Faser (15) mit einer ersten Periode in Schwingungen zu versetzen; **gekennzeichnet durch**
eine zweite Antriebseinheit (15B), die dafür geeignet ist, die Faser (15) mit einer zweiten Periode in Schwingungen zu versetzen,
wobei die mithilfe der zweiten Antriebseinheit (15B) erzeugte Amplitude größer ist als die mithilfe der ersten Antriebseinheit (16) erzeugte Amplitude.

2. Laserablationsvorrichtung (10, 30, 40) nach Anspruch 1,
wobei die erste Antriebseinheit (16) näher dem distalen Ende der Faser (15) vorgesehen ist als die zweite Antriebseinheit (15B); und
die zweite Periode länger als die erste Periode ist.

3. Laserablationsvorrichtung (10, 30, 40) nach Anspruch 1 oder 2, wobei die erste Antriebseinheit (16) näher dem distalen Ende der Faser (15) vorgesehen ist als die zweite Antriebseinheit (15B); und
eine Länge der zweiten Periode n-mal (n ist eine Ganzzahl) eine Länge der ersten Periode ist.

4. Laserablationsvorrichtung (10, 30, 40) nach einem der Ansprüche 1 bis 3,
wobei die Faser (15) dafür geeignet ist, Drehbewegungen mithilfe der ersten Antriebseinheit (16) und der zweiten Antriebseinheit (15B) durchzuführen; und
die Anzahl von Drehungen der Faser (15) aufgrund der ersten Antriebseinheit (16) höher ist als die Anzahl von Drehungen der Faser (15) aufgrund der zweiten Antriebseinheit (15B).

5. Laserablationsvorrichtung (10, 30, 40) nach einem der Ansprüche 1 bis 4, wobei die Faser (15) dafür geeignet ist, eine Resonanzbewegung durchzuführen.

6. Laserablationsvorrichtung (10, 30, 40) nach einem der Ansprüche 1 bis 3, wobei
die Faser (15) dafür geeignet ist, eine Rasterabtastung durchzuführen.

7. Laserablationsvorrichtung (10, 30, 40) nach einem der Ansprüche 1 bis 5, wobei
die Faser (15) dafür geeignet ist, eine Spiralabtastung durchzuführen.

8. Laserablationsvorrichtung (10, 30, 40) nach einem der Ansprüche 1 bis 7, die ferner eine oder mehrere Antriebseinheiten umfasst, die dafür geeignet sind, die Faser (15) periodisch in Schwingungen zu versetzen.

## Revendications

1. Cathéter d'ablation par laser (10 ; 30 ; 40) comprenant :
une source de lumière (17a) adaptée pour émettre une lumière laser pour cautériser une région affectée ;
une partie d'insertion (11) configurée pour être insérée dans le corps et qui est un long conduit de tuyau flexible ;
une fibre (15) qui est disposée dans la partie d'insertion (11) et adaptée pour guider la lumière laser émise à partir de la source de lumière (17a) pour rayonner la lumière laser à partir de l'extrémité distale de la partie d'insertion (11) ;
une première unité d'entraînement (16) qui est disposée sur la fibre (15) et est adaptée pour faire vibrer la fibre (15) avec une première période ;
**caractérisé par** :
une seconde unité d'entraînement (15B) qui est adaptée pour faire vibrer la fibre (15) avec une seconde période,
l'amplitude produite par la seconde unité d'entraînement (15B) étant plus grande que l'amplitude produite par la première unité d'entraînement (16).

2. Dispositif d'ablation par laser (10 ; 30 ; 40) selon la revendication 1,
dans lequel la première unité d'entraînement (16) est disposée plus près de l'extrémité distale de la fibre (15) que la seconde unité d'entraînement (15B) ; et
la seconde période est plus longue que la première période.

3. Dispositif d'ablation par laser (10 ; 30 ; 40) selon la revendication 1 ou 2,
dans lequel la première unité d'entraînement (16) est disposée plus près de l'extrémité distale de la fibre (15) que la seconde unité d'entraînement (15B) ; et
une longueur de la seconde période est n fois (n est un entier) une longueur de la première période.

4. Dispositif d'ablation par laser (10 ; 30 ; 40) selon l'une des revendications 1 à 3,
dans lequel la fibre (15) est adaptée pour réaliser des mouvements de rotation par la première unité d'entraînement (16) et par la seconde unité d'entraînement (15B) ; et
le nombre de rotations de la fibre (15) dues à la première unité d'entraînement (16) est plus grand que le nombre de rotations de la fibre (15) dues à la seconde unité d'entraînement (15B).

5. Dispositif d'ablation par laser (10 ; 30 ; 40) selon l'une des revendications 1 à 4, dans lequel la fibre (15) est adaptée pour réaliser un mouvement résonant.

6. Dispositif d'ablation par laser (10 ; 30 ; 40) selon l'une des revendications 1 à 3, dans lequel la fibre (15) est adaptée pour réaliser un balayage de trame.

7. Dispositif d'ablation par laser (10 ; 30 ; 40) selon l'une des revendications 1 à 5, dans lequel la fibre (15) est adaptée pour réaliser un balayage en spirale.

8. Dispositif d'ablation par laser (10 ; 30 ; 40) selon l'une des revendications 1 à 7, comprenant en outre une ou plusieurs unités d'entraînement qui sont adaptées pour faire vibrer la fibre (15) de manière périodique.
